# EUROPÄISCHE PATENTSCHRIFT

(11) **EP 3 143 221 B1**
(45) Veröffentlichungstag und Bekanntmachung des Hinweises auf die Patenterteilung: **31.01.2018**
(21) Anmeldenummer: 15734053.0
(22) Anmeldetag: 11.05.2015
(51) Int. Cl.: E04F 11/18, E04H 12/22, F16C 11/06

(54) **VERSTELLBARER GELÄNDERPFOSTEN**
ADJUSTABLE GUARD RAIL POST
POTEAUX RÉGLABLES DE GARDE-CORPS

(30) Priorität: 14.05.2014 AT 3502014
(43) Veröffentlichungstag der Anmeldung: 22.03.2017
(73) Patentinhaber: Holzbauer, Eduard, 9470 St. Paul (AT)
(72) Erfinder: Holzbauer, Eduard, 9470 St. Paul (AT)
(74) Vertreter: Beer & Partner Patentanwälte KG
(86) Internationale Anmeldenummer: PCT/AT2015/000069
(87) Internationale Veröffentlichungsnummer: WO 2015/172167

(56) Entgegenhaltungen:
- DE-U1- 20 014 014
- FR-A1- 2 591 635
- GB-A- 2 235 937
- US-A- 5 458 428
- US-A1- 2008 277 640
- US-A1- 2009 272 957

## Beschreibung

Die Erfindung betrifft einen **verstellbaren Geländerpfosten,** insbesondere zur Anbringung auf Balkonplatten und dergleichen.

US 2008/0277640 offenbart einen mittels eines Kugelgelenks verstellbaren Geländerpfosten.

Geländerpfosten, die auf Balkonplatten angebracht werden, sind in unterschiedlichen Ausführungen bekannt. Als nachteilig erweist sich, dass Geländerpfosten, die gekauft oder angefertigt werden, nicht dem Gefälle der Balkonplatten angepasst und starr sind. Egal ob Balkonplatten, Terrassenböden, Stiegenaufgänge, Brüstungen und dergleichen, sind unterschiedlich. Des Öfteren müssen Unebenheiten nachbearbeitet werden. Hier treten bei der Montage der Geländerpfosten oder des Handlaufs große Schwierigkeiten und Zeitbeanspruchung auf. Auch für das Auge sieht es nicht schön aus, wenn ein Geländerpfosten nach vorne der andere nach hinten, links oder rechts neigt.

Der Erfindung liegt demnach die Aufgabe zugrunde, einen Geländerpfosten der eingangs erwähnten Art so zu verbessern, dass die oben erwähnten Nachteile nicht zu tragen kommen.

Das wird erfindungsgemäß durch die kennzeichnenden Merkmale des Anspruch 1 erreicht. Weitere vorteilhafte Ausgestaltungen, werden gemäß den Unteransprüchen vorgeschlagen.

Die Erfindung wird nun unter Bezugnahme auf ein Ausführungsbeispiel, welches in der Zeichnung schematisch dargestellt ist, weiter erläutert.

Es ist Ziel, Gefälle, Steigungen und Unebenheiten entgegen zu wirken. Die vielen unterschiedlichen Untergründe verursachen hohe Herstellungs- und Montage Kosten. Da viele Unebenheiten erst bei der Montage entdeckt werden, wird mehr Zeit benötigt. Es ist daher vorteilhaft, die Geländerpfosten so anzufertigen, dass derartige Probleme mit zwei Halbschalen gelöst werden. Ziel ist es daher die Geländerpfosten in den drei Freiheitsgraden eines Kugelgelenkes einstellen und für verschiedene Bedarfe verwenden zu können.

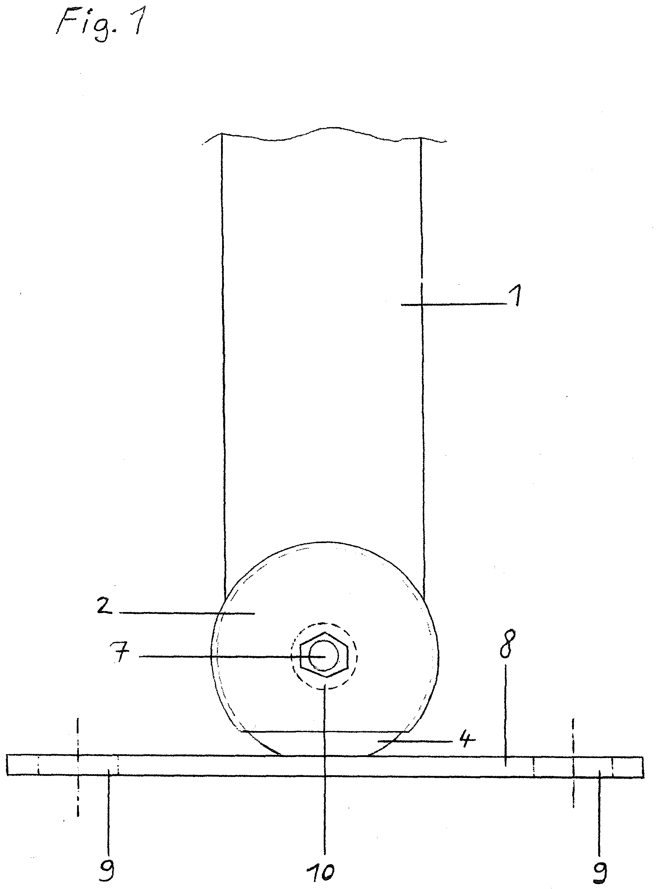

Fig.1 zeigt die Vorderansicht des verstellbaren Geländerpfostens und

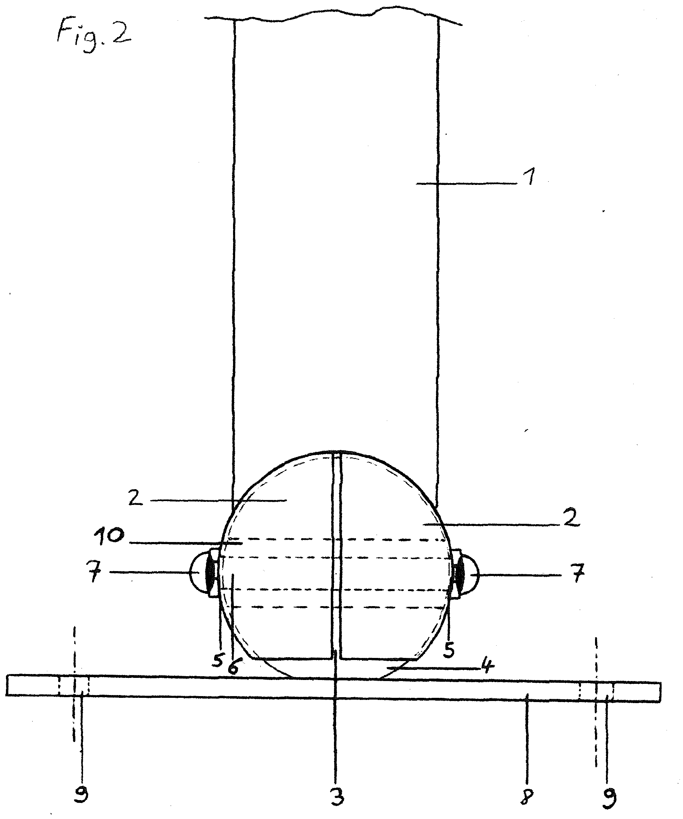

Fig.2 eine Seitenansicht

Eine mit der Ankerplatte 8 fix verbundene Kugel 4 ist teilweise von zwei Halbschalen 2 umgriffen, welche sich unter Ausbildung eines Spaltes 3 nicht berühren. Die Halbschalen 2 enthalten konzentrische, parallel zur Ankerplatte 8 verlaufende Bohrungen 5, 10. Eine die Kugel 4 und die Halbschalen 2 durchsetzende Gewindestange 6 wird durch Anziehen der Muttern 7 die Halbschalen 2 an die Kugel 4 gepresst und somit der Geländerpfosten 1 gehalten.

## Patentansprüche

1. Verstellbarer Geländerpfosten, insbesondere zur Anbringung auf unebenen Untergründen, **dadurch gekennzeichnet, dass** an dem Geländerpfosten (1) im unteren Bereich zwei Halbschalen (2) angeordnet sind, welche eine mit einer Ankerplatte (8) fix verbundene Kugel (4) unter Ausbildung eines Spaltes (3) teilweise umgreifen, wobei die Kugel (4)und die Halbschalen (2) konzentrische, parallel zur Ankerplatte (8) verlaufende Bohrungen (5,10) aufweisen und eine die Kugel (4) und die Halbschalen (2) durchsetzende Gewindestange (6) mit Muttern (7) vorgesehen ist.

2. Geländerpfosten nach Anspruch 1, **dadurch gekennzeichnet, dass** die Kugel (4) und deren teilweise umgreifenden Halbschalen (2) unterschiedliche Bohrungsdurchmesser (5,10) besitzen.

3. Geländerpfosten nach Anspruch 1 oder 2, **dadurch gekennzeichnet, dass** an die Kontur der Halbschalen (2) die Innenflächen der Muttern (7) angepasst sind.

4. Geländerpfosten nach einem der Ansprüche 1 bis 3 **dadurch gekennzeichnet, dass** die Öffnungen in der Ankerplatte (8) als Langlöcher (9) ausgebildet sind.

5. Geländerpfosten nach einem der Ansprüche 1 bis 4 **dadurch gekennzeichnet, dass** die Kugeloberfläche (4) und die Innenseiten der Halbschalen (2) gerändelt sind.

## Claims

1. Adjustable railing post, in particular for attachment to uneven underlying surfaces, **characterized in that** two half-shells (2) are arranged on the railing post (1) in the lower region, which partially enclose a ball (4) fixedly connected to an anchor plate (8) while forming a gap (3), wherein the ball (4) and the half-shells (2) have concentric boreholes (5, 10) extending parallel to the anchor plate (8) and a threaded rod (6), which penetrates the ball (4) and the half-shells (2), having nuts (7) is provided.

2. Railing post according to Claim 1, **characterized in that** the ball (4) and the half-shells (2) which partially enclose it have different borehole diameters (5, 10).

3. Railing post according to Claim 1 or 2, **characterized in that** the inner faces of the nuts (7) are adapted to the contour of the half-shells (2).

4. Railing post according to any one of Claims 1 to 3, **characterized in that** the openings in the anchor plate (8) are formed as oblong holes (9).

5. Railing post according to any one of Claims 1 to 4, **characterized in that** the ball surface (4) and the inner sides of the half-shells (2) are knurled.

## Revendications

1. Poteau de rambarde réglable, en particulier pour une installation sur un support inégal, **caractérisé en ce que** sont disposés dans la partie inférieure du poteau de rambarde (1) deux demi-coques (2) qui entourent partiellement une boule (4) fixée à une plaque d'ancrage (8) en formant un interstice (3), dans lequel la boule (4)et les demi-coques (2) présentent des perçages (5, 10) concentriques et parallèles à la plaque d'ancrage (8) et il est prévu une tige filetée (6) qui traverse la boule (4) et les demi-coques (2) avec des écrous (7).

2. Poteau de rambarde selon la revendication 1, **caractérisé en ce que** la boule (4) et les demi-coques (2) qui l'entourent en partie présentent des diamètres de perçage (5, 10) différents.

3. Poteau de rambarde selon la revendication 1 ou 2, **caractérisé en ce que** les surfaces intérieures des écrous (7) sont adaptées au contour des demi-coques (2).

4. Poteau de rambarde selon l'une des revendications 1 à 3, **caractérisé en ce que** les ouvertures dans la plaque d'ancrage (8) sont réalisées comme des trous oblongs (9).

5. Poteau de rambarde selon l'une des revendications 1 à 4, **caractérisé en ce que** la surface de la boule (4) et les faces intérieures des demi-coques (2) sont moletées.
